(19) Europäisches Patentamt / European Patent Office / Office européen des brevets

(11) **EP 2 169 400 A1**

(12) **EUROPEAN PATENT APPLICATION**

(43) Date of publication:
**31.03.2010 Bulletin 2010/13**

(51) Int Cl.:
*G01N 33/00* (2006.01)     *G01N 27/22* (2006.01)
*B81B 3/00* (2006.01)

(21) Application number: **08165183.8**

(22) Date of filing: **25.09.2008**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(71) Applicant: **IEE INTERNATIONAL ELECTRONICS &
ENGINEERING S.A.
6468 Echternach (LU)**

(72) Inventor: **Boulbitch, Alexei
54295, Trier (DE)**

(74) Representative: **Office Freylinger
P.O. Box 48
8001 Strassen (LU)**

(54) **Hydrogen sensor**

(57) A hydrogen sensor comprises at least one reactive layer (15,16) spanning at least along a first direction between two supporting points (13). The at least one reactive layer comprises an hydrogen-absorbent material (16), which has a capability to expand upon absorption of hydrogen. The sensor further comprises an assembly (12) for detecting a deflection of said reactive layer, said deflection being caused by an expansion of said hydrogen-absorbent material in said first direction.

**Fig. 5C**

EP 2 169 400 A1

**Description**

**Technical field**

This invention relates generally to a gas sensor for detecting the presence of hydrogen in gas streams or in the environment, and for measuring the amount of such hydrogen. More particularly, the present invention relates to a hydrogen sensor to be used for the detection of hydrogen gas of relatively low concentration leaking from various devices such as fuel cell for automobile, fuel cell for household use, and the like.

**Background Art**

Wherever hydrogen is handled, e.g. in power stations, fuel cells, for safety control during recharging accumulators, especially in air and space crafts and in submarines, as well as in the buffer batteries of nuclear power stations, there is a need for hydrogen sensors which can reliably detect the presence of hydrogen in the environment. As hydrogen is considered as an alternative to oil-derivative fuels in the automotive industry, there is an increasing need to have a reliable control of the hydrogen concentration in many points within the vehicle as well as inside garages and tanking places.

Because of the high flammability of hydrogen gas ($H_2$) in air, there is a need for detecting hydrogen at levels below its lower explosive limit (about 4% at 25°C). A conventional approach to detect $H_2$ is e.g. based upon the resistance increase of palladium (Pd) taking place when hydrogen diffuses into the metal and thereby gives rise to palladium hydride having higher volume and lower conductivity than pure palladium.

It is generally accepted that although the number of different hydrogen detectors is huge, there is no detector that would simultaneously satisfy all requirements of say, automotive industry. Indeed, the known sensors are either unsuitable for low density detection of hydrogen or suffer from a low response time or short lifetime. In addition most of these sensors are expensive.

**Technical problem**

The aim of the present invention is to offer an inexpensive, sensitive hydrogen sensor for measuring small hydrogen concentration in air with a fast reaction time. This object is achieved by a hydrogen sensor as claimed in claim 1.

**General Description of the Invention**

In order to achieve the above-mentioned object, the present invention proposes a hydrogen sensor, comprising at least one reactive layer spanning at least along a first direction between two supporting points. The at least one reactive layer comprises an hydrogen-absorbent material, which has a capability to expand upon absorption of hydrogen. The sensor further comprises an assembly for detecting a deflection of said reactive layer, said deflection being caused by an expansion of said hydrogen-absorbent material in said first direction.

Many metals, for instance palladium, niobium, vanadium or alloys thereof (e.g. with gold or silver), yttrium, $LaNi_5$ and others exhibit a capability to absorb large amounts of hydrogen. The sensing mechanism used in the present invention is based on the ability of these materials to adsorb and accumulate atomic hydrogen in a hydrogen atmosphere, thus resulting in their dilatation. In a reactive layer produced with such a hydrogen-absorbent material the hydrogen absorption results (along with other effects) in a lateral dilatation of the hydrogen-absorbent material. The reactive layer is fixed on at least a part of its boundary to the two supporting points in such a way, that the reactive layer spans at least along a first direction between two supporting points. Absorption of hydrogen in the hydrogen-absorbent material of the reactive layer causes among others an expansion of the hydrogen-absorbent material of the reactive layer, which results in a deflection of the reactive layer. This deflection may be detected and its amplitude may be measured by a variety of approaches.

The following calculations constitute a theoretical basis of the present invention.

**1. Dilatation followed hydrogen absorption**

Different metals possess different ability to accumulate hydrogen characterized by the so-called solubility diagrams of hydrogen. The latter represent graphs of concentration of the hydrogen dissolved in the metal in question versus partial pressure of the hydrogen in air. The concentration of the dissolved hydrogen is usually given with respect to the concentration of the matrix atoms, $x = n_H/n_{Me}$, where the subscripts "H" and "Me" stand for hydrogen and metal correspondingly. Solubility diagrams may be found in numerous publications and reviews. Here we point out only the classical paper reporting solubility of hydrogen in palladium, the classical hydrogen-absorbent material, Simons, J. W. & Flanagan, T. B. Absorption isotherms of hydrogen in the .alpha.-phase of the hydrogen-palladium system. Journal of Physical Chemistry 69, 3773-3781 (1965) as well as the book "Hydrogen in metals I and II" (eds. Alefeld, G. & Völkl, J.)

(Springer-Verlag Berlin, Heidelberg and New York, 1978). By direct analysis of these data we confer that the solubility of hydrogen at low pressures of the gaseous hydrogen and below any hydrogen-induced phase transition of metal is perfectly described by the following simple expression:

$$x = \frac{n_H}{n_{Me}} = k_1 \sqrt{p_H} + k_2 p_H \qquad (1)$$

where $p_H$ is the partial pressure of hydrogen in air and the parameters $k_1$ (bar$^{-1/2}$) and $k_2$(bar$^{-1}$) are expressed as:

$$k_{1,2} = A_{1,2} \exp\left(\frac{B_{1,2}}{T}\right) \qquad (2)$$

Here T is the temperature (measured in grad K), $A_{1,2}$ and $B_{1,2}$ are material constants specific for each metal. In the case of palladium for instance, $A_1$=1.33×10$^{-3}$ bar$^{-1/2}$, $B_1$=1170.5 K$^{-1}$, $A_2$=1.78×10$^{-5}$ bar$^{-1}$, $B_2$=2736.3 K$^{-1}$.

Further it is known that dissolution of hydrogen causes the metal dilatation, the volume increase caused by dissolution of one hydrogen atom being the same for all cubic metals equal to 2.9 Å$^3$ and is of the same order in other cases (see the book "Hydrogen in metals I" cited above). This leads to the conclusion that the dilatation of the metal caused by hydrogen is described as

$$\frac{\Delta V}{V} = 3\gamma\, n_H \qquad (3)$$

where V is the metal volume, $\Delta V$ is its increment, $\gamma$ is a constant. For cubic metals $\gamma$=2.9×10$^{-30}$ m$^3$ (see Boulbich, A. A. J. Alloys Comp. 196, 29 (1993)). In the case of a metal stripe its lateral extension, $\varepsilon = \Delta l / l_0$ (where $l_0$ is the stripe length, and $\Delta l$ is its increment due to the hydrogen) is expressed as

$$\varepsilon = \gamma\, n_H \qquad (4)$$

**2. Hydrogen-induced deflection or buckling of a metal plate**

If a metal plate is fixed somehow in its boundaries the lateral extension described above causes in-plane stresses that may further cause the plate buckling or deflecting. Here we illustrate this statement by the specific case of the plate in a form of a stripe, length 2$l_0$, with a, clamped in its ends. Behavior of such a stripe may be considered within the theory of buckling of rods (see for example, Volmir, A. S. Stability of elastic systems (in Russian) (State publisher of literature in Physics and Mathematics, Moscow, 1963). The solution is defined by three parameters: the buckling amplitude, f, as well as the parameters k and m. Parameter k is the ratio of a lateral force acting along the stripe to its bending modulus. For details one can address the book of Volmir cited above. However, since neither this force nor the bending modulus is of interest here, parameter k will not be discussed more precisely. Parameter m is expressed in terms of k and f as follows:

$$m = k^2 f^2 / 4 \qquad (5)$$

Solution of the problem requires finding of the deflection amplitude, f, of the stripe caused by its hydrogen-induced elongation, $\varepsilon = \Delta l / l_0$. The exact implicit solution of the above problem has the following form

$$\varepsilon = \frac{2K(m) - 2E(m)}{2E(m) - K(m)}$$

$$kl_0 = 2E(m) - K(m) \qquad (6)$$

$$f = \frac{2l_0\sqrt{m}}{k}$$

Here $K(m)$ and $E(m)$ are the elliptic integrals of the first and second kind correspondingly defined as follows:

$$K(m) = \int_0^{\pi/2}\left(1 - m\sin^2\varphi\right)^{-1/2}d\varphi \text{ and } E(m) = \int_0^{\pi/2}\left(1 - m\sin^2\varphi\right)^{1/2}d\varphi \text{ (see Abramowitz, M. \& Stegun, I. A.}$$

Handbook of Mathematical Functions with formulas, graphs and mathematical tables. (National Bureau of Standards, 1964) and accounts for arbitrary (rather than only small) deflections of the stripe.

Expressions (6) give an implicit exact solution of the problem, since (at least in principle) the first equation (6) yields $m=m(\varepsilon)$, then the second one defines $k=k(m)$ and thus $k=k(\varepsilon)$. After that the amplitude, $f$, is given by the third equation (6). An exact explicit solution of this problem cannot be found. However, the problem admits a numeric solution which can be then approximated by the expression

$$f = l_0\left(1.26\sqrt{\varepsilon} + 0.12\varepsilon\right) \qquad (7)$$

valid for $0 \leq \varepsilon \leq 0.1$

Combining this solution with (1, 2) and (4) one finds the deflection amplitude of the device depending upon the partial pressure of the hydrogen in ambient air as well as upon temperature. Finally, one may use the relation

$$p_H / p = x_H = N_H / N_{Air} \qquad (7a)$$

where $p_H$ is the partial hydrogen pressure, $p$ is the atmospheric pressure which we take to be strictly equal to 1 bar, $x$ is the concentration defined above, $N_H$ is the number of the hydrogen molecules in air and $N_{Air}$ is the number of all molecules. This enables to obtain dependence of the sensitivity upon the hydrogen concentration, $x$, and the temperature.

It will be noted that due to the arrangement and the fixation of the reactive layer on two supporting points, the absorption of hydrogen in the hydrogen-absorbent material is followed by a considerable strain in the layer of the hydrogen-absorbent material which results in a high amplitude of the deflection of the reactive layer. Accordingly, if the hydrogen-absorbent material is suitably chosen, the deflection of the reactive layer is quite distinctive even for small amounts of hydrogen absorbed. This means that the sensor of the present invention may be used for the detection of very small concentrations of hydrogen in the air.

In a possible embodiment of the invention, said reactive layer comprises a lamina of said hydrogen-absorbent material, and wherein said lamina of said hydrogen-absorbent material is fixed to said supporting points. In a more preferred embodiment however, the reactive layer comprises a flexible carrier layer and a layer of hydrogen-absorbent material applied to said flexible carrier layer, and wherein said flexible carrier layer is fixed to said supporting points.

The carrier layer may be made from any material which allows considerable bending deformations, such as e.g. a metal or preferably a polymer such as e.g. polyethylene, polyethylene terephthalate or any other polymer suitable for the production purpose. The asymmetry of the bi-layered reactive layer helps to guide the deflection of the reactive layer in the desired direction. In fact, as the hydrogen-absorbent material experiences the lateral dilatation while the carrier layer does not, the bending takes place such that the hydrogen-absorbent material layer is always on the convex side. Furthermore the bi-layered structure of the reactive layer simplifies the manufacture and the handling of the hydrogen sensor.

In choosing a suitable carrier layer material, one preferably makes sure that the following inequality holds $E_{HAM}h_{HAM} > E_P h_P$, where $E_{HAM}$ and $E_P$ are the Young's moduli of the hydrogen-absorbent material and of the polymer substrate while $h_{HAM}$ and $h_P$ are the respective thicknesses of the two layers. For example, The Young's modulus of palladium is 121 Gpa, the one of polyethylene (PE) is 172 MPa and the one of polyethylene terephthalate (PET) is about 3 GPa. Thus, if the bi-layered reaction layer is produced of the pair polyethilene and palladium, and the thickness of the palladium layer, $h_{Pd}$ is 1 $\mu$m, one finds $h_P < h_{Pd}E_{Pd}/E_P \approx 700$ $\mu$m. If the PET is used for such a purposes one finds $h_P < 40\mu$m.

For applying the hydrogen-absorbent material to said flexible carrier layer one may use any suitable method of deposition such as spin-coating, deposition from the gas phase etc. However one particularly preferred method consists of printing of the hydrogen-absorbent material on top of the carrying film, e.g. by screen printing or ink jet printing. The thickness of the layer should not exceed the value h~(Dt)1/2, where D is the diffusion coefficient of the hydrogen in the corresponding hydrogen-absorbent material, while t is time interval of the order of the time of the sensor reaction. For example, automotive applications require the reaction time t below 1 second. The diffusion coefficient of palladium is D~10-11 m2/s yielding h below 1 mm.

As some of the hydrogen-absorbent material exhibit degradation of their surfaces (such as e.g. corrosion or oxidation), it is preferred that said reactive layer further comprises a protective layer, said protective layer being arranged on the hydrogen-absorbent material. In the case of yttrium as hydrogen-absorbent material, it is for instance preferred to have a thin protective layer of palladium over the layer of yttrium. The protective layer may have any thickness, provided it is much smaller than h determined above. Because of high prices of the materials in question the thicknesses in the range from few tens to few hundreds nanometers are preferential. This layer fulfills solely a protective role and may be necessary in some applications and unnecessary in others. For example, if the hydrogen-sensitive layer is formed by palladium such a protective layer is not necessary.

The shape of the reactive layer may be freely chosen, provided that the shape enables one to fix the boundary of the said sheet in such a way that the sheet lateral dilatation gives rise to its buckling. In a preferred embodiment said at least one reactive layer has a rectangular form with two shorter sides and two longer sides, and wherein each of said shorter sides of said reactive layer is fixed to one of said supporting points. An alternative configuration would be e.g. a disk-shaped reactive layer mounted and fixed on an annular supporting structure. The fixing of the reactive layer to the supporting points may be achieved by gluing, welding or clamping or any other suitable method.

The amplitude of the deflection of the reactive layer may be detected in different ways. In a possible embodiment, the assembly for detecting a deflection of said reactive layer may comprise an optical system for determining reflection of a light beam at the deflected reactive layer. As an example of such an optical system, a micro-mirror may be arranged on reactive layer and a light source (such as laser, lamp, LED etc.) along with the light detectors are arranged on a housing of the device. Bending of the membrane will cause a deflection of the light beam registered by the said light detectors.

In a different embodiment, the deflection of the reactive layer may be detected by the establishment of an electrical contact. In one variant of this embodiment, the assembly for detecting a deflection of said reactive layer comprises an electrode structure arranged in facing relationship with said hydrogen-absorbent material at a certain distance from said hydrogen-absorbent material and an associated electric circuit for detecting if an electrical contact is established between said hydrogen-absorbent material and said electrode structure due to the deflection of said reactive layer.

It will be noted, that the electrode structure may preferably be applied to an electrode carrier layer, which is arranged in facing relationship with said hydrogen-absorbent material at a certain distance from said reactive layer by means of a spacer. The spacer may in this embodiment also act as supporting element for the reactive layer. The electrode carrier layer, the spacer and the reactive layer are then preferably laminated together in order to obtain a well defined configuration of the sensor element.

In another variant said assembly for detecting a deflection of said reactive layer comprises a first electrode structure arranged on said reactive layer, a second electrode arranged in facing relationship with said first electrode structure at a certain distance from said first electrode structure, and an associated electric circuit for detecting if an electrical contact is established between said first electrode structure and said second electrode structure due to the deflection of said reactive layer. Here again the first electrode structure is preferably applied to an electrode carrier layer, which is arranged in facing relationship with said hydrogen-absorbent material at a certain distance from said reactive layer by means of a spacer and laminated together to form a compact sensor element.

In a preferred embodiment of the invention, the bending of the reactive layer is registered by the measurement of the capacitance of the sensor. In this embodiment, the assembly for detecting a deflection of said reactive layer comprises an electrode structure arranged at a certain distance from said hydrogen-absorbent material and an associated electric circuit for determining a variation of capacitance between said hydrogen-absorbent material and said electrode structure due to the deflection of said reactive layer. Alternatively the assembly for detecting a deflection of said reactive layer comprises a first electrode structure arranged on said reactive layer, a second electrode arranged at a certain distance from said first electrode structure, and an associated electric circuit for determining a variation of capacitance between said first electrode structure and said second electrode structure due to the deflection of said reactive layer. Again the electrode structure or the first electrode structure is preferably applied to an electrode carrier layer, which is arranged at a certain distance from said reactive layer by means of a spacer and laminated together to form a compact sensor element.

The capacitance of the two electrodes may be measured directly by using a bridge scheme. Functioning of the said device is illustrated by the following calculations.

The capacitance of a strip shaped device is expressed as

$$\frac{C}{C_0} = \int_0^{l0} \frac{dx}{1 \pm y(x)/d_0} \tag{8}$$

where C is the actual capacity of the sensor, $d_0$ is the height of the spacers, i.e. the distance between the two electrodes, when the reactive layer is plane, $C_0$ is the sensor capacitance in this state, $y(x)$ is the function defining the shape of the reactive layer stripe, the sign "+" corresponds to the case of the outward buckling, while "-" is valid in the inward bending case. Using a change of variables that has been proposed in the book [6] one brings (8) to the expression

$$K = \frac{|C - C_0|}{C_0} \approx \left| \frac{2J_1(\varepsilon, z) - J_2(\varepsilon, z)}{1.57 - 1.08\varepsilon} - 1 \right| \tag{9}$$

referred to as the "sensitivity". Here the following notations are utilized:

$$J_1(\varepsilon, z) = \int_0^{\pi/2} \frac{\Delta(\varepsilon, \varphi)}{1 \pm z(1.26\sqrt{\varepsilon} + 0.12\varepsilon) \times \cos\varphi} d\varphi$$

$$J_2(\varepsilon, z) = \int_0^{\pi/2} \frac{1}{[1 \pm z(1.26\sqrt{\varepsilon} + 0.12\varepsilon) \times \cos\varphi] \times \Delta(\varepsilon, \varphi)} d\varphi$$

$$\Delta(\varepsilon, \varphi) = \sqrt{1 - (0.99\varepsilon + 0.96\varepsilon^2) \times \sin^2 \varphi} \; ; \qquad z = l_0 / d_0$$

As in the previous case the signs "+" and "-" in the integrals met in Eq. (9) are valid in the cases of the outward and inward buckling correspondingly. It should be noted that in the case of the inward buckling the integrals may diverge in a certain range of values of the parameters. This divergence reflects the situation when the electrodes of the sensor first touch one another. After this event the equation (9) is not valid any more. The integration (9) should be therefore, performed only for those values of the parameters for which the integrals $J_1$ and $J_2$ converge.

Further combination of (9) with (1, 2) and (4) and (7a) yields dependence of the sensitivity (9) upon the partial pressure of hydrogen in air as well as upon temperature and the aspect ratio parameter z. The integrals can easily be calculated numerically at various values of the parameters.

It will be noted, that according to one aspect of the present invention the outward bending may be used in order to be able to measure the hydrogen content in a wide range of the concentrations while the inward buckling may be used in order to increase the device sensitivity in a preferred range of the hydrogen concentrations.

It will be noted that in the case of capacitive registration of the reactive layer deflection, the sensor should preferably be shielded from influences of external electric field. This shielding may be provided by placing the whole device inside a grounded metallic housing in which orifices are provided in order to open the sensitive element for a free access of the ambient gas. This for example, may be achieved by a housing at least partially produced out of a grounded wire mesh.

According to a further aspect of the present invention a pump is provided in order to force the ambient air to fast move to the hydrogen-sensitive electrode thus, decreasing the contribution to the reaction time of the sensor which is related to the exchange of the gas at the sensitive element.

According to a still more preferential embodiment the grounded hydrogen-sensitive electrode itself represents at least a part of the electrically screening housing.

**Brief Description of the Drawings**

Further details and advantages of the present invention will be apparent from the following detailed description of several not limiting embodiments with reference to the attached drawings, wherein:

Fig. 1 shows the solubility diagrams of hydrogen in palladium;

Fig. 2 schematically shows a thin buckled stripe clamped at its both ends;

Fig. 3 represents a numerical solution of equations (6) along with its analytical approximation.

Fig. 4 shows the graph of the deflection amplitude of the bi-layer stripe versus the concentration of the hydrogen in air

Fig. 5 (a-e) shows schematic designs of various embodiments of the outward- and inward buckling capacitive hydrogen sensors.

Fig. 6 Schematically shows two electrodes of the capacitive hydrogen sensor during the outward buckling (a) and the inward buckling (b).

Fig. 7. (a-c) shows the dependence of the read-out of the read-out of the capacitive sensor exhibiting the outward buckling at three typical values of the aspect ratio and four temperatures.

Fig. 8 (a, b) show the same under the inward buckling.

**Description of Preferred Embodiments**

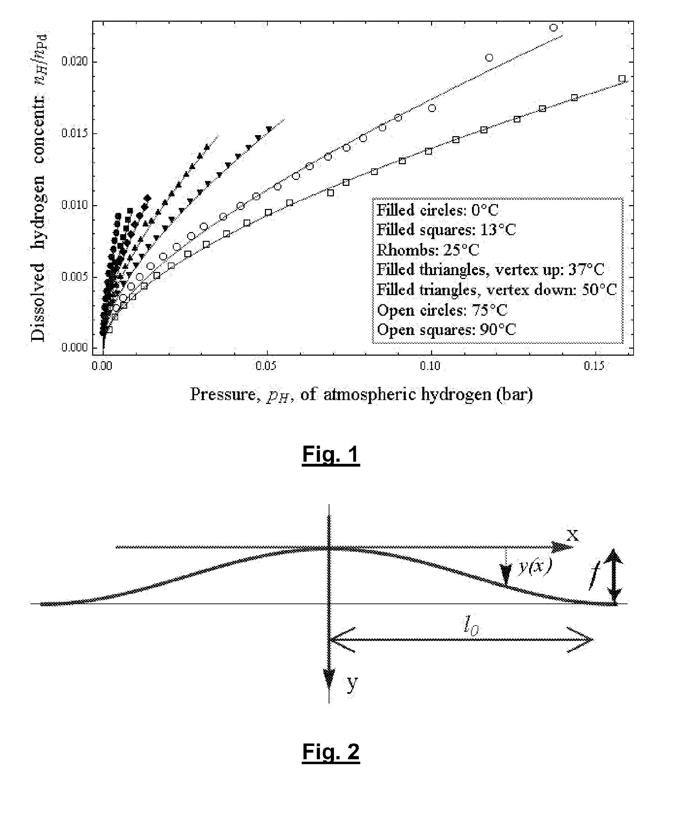

Figure 1 shows the solubility diagrams of hydrogen in palladium. Experimental data on solubility diagrams are taken from the paper Simons, J. W. & Flanagan, T. B. Absorption isotherms of hydrogen in the .alpha.-phase of the hydrogen-palladium system. Journal of Physical Chemistry 69, 3773-3781 (1965). Solid lines show concentration of the dissolved hydrogen described by eq. (1, 2)

Figure 2 shows a schematic view of the buckling plate clamped at its both ends. The initial position of the plate is shown by the dashed line.

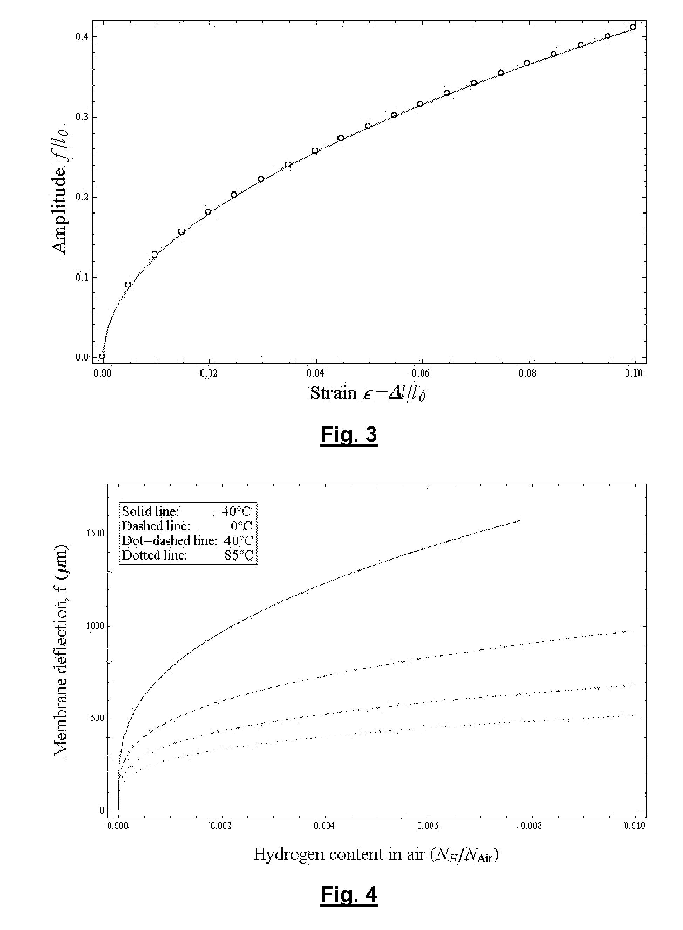

Figure 3 displays a numeric solution of the system (6) (circles) yielding dependence of the amplitude of the stripe deflection versus its extension, along with its approximation with the analytical expression (7) (the solid line).

Figure 4 exhibits the deflection amplitude of the stripe with the half-length $l_0$=1 cm versus hydrogen concentration in air at temperatures characteristic for automotive applications. Solid line corresponds to the temperature -40°C, dashed line: 0°C, dotted line: 40°C and dot-dashed line: 85°C.

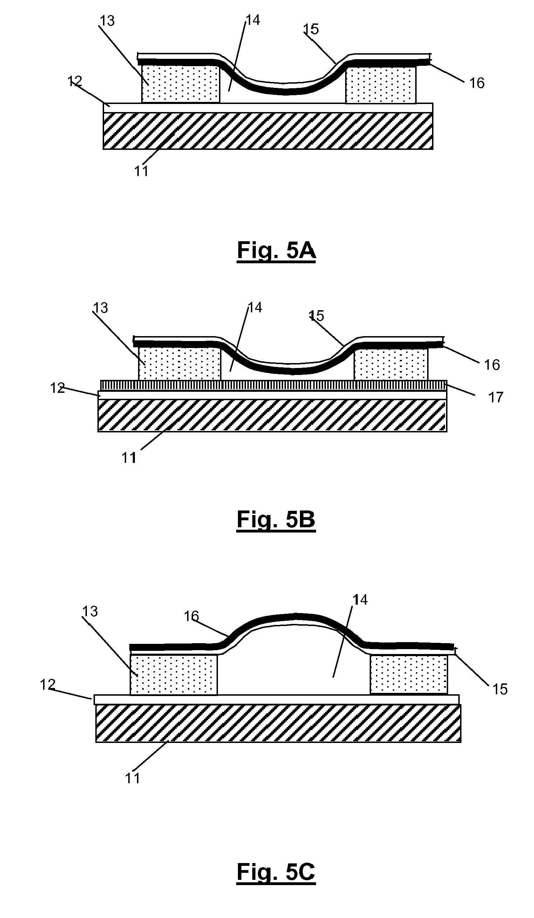

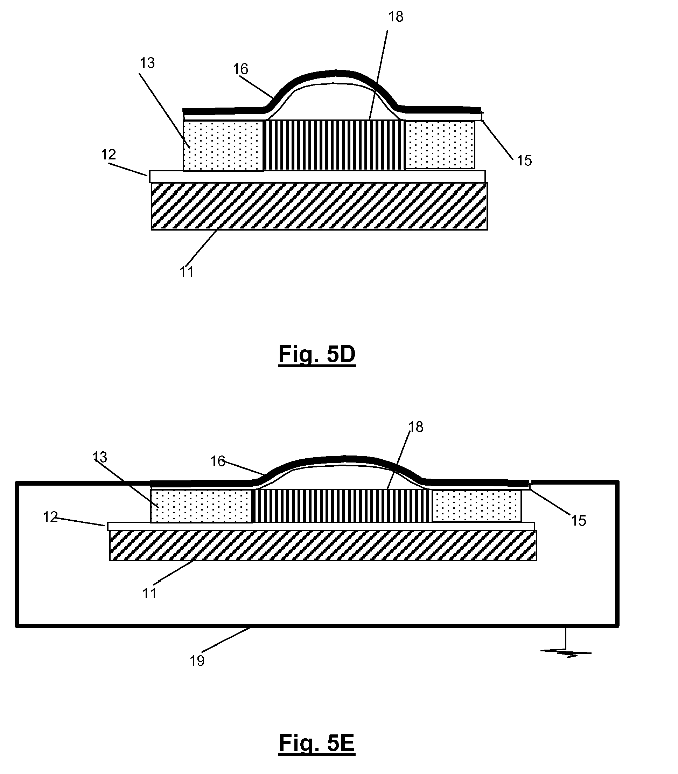

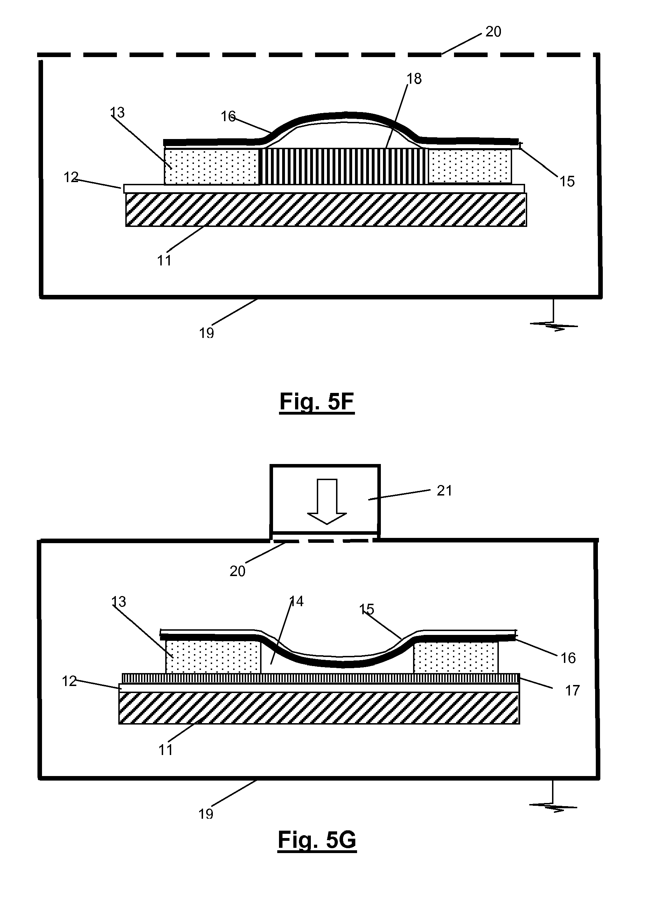

Figure 5 (a-g) shows various embodiments of the capacitive hydrogen sensor. The sensor cell consists of the substrate 11, first electrode layer 12, spacers 13, recess 14, bilayer film 15, 16, where 15 shows the polymer film, while 16 indicates the hydrogen-absorbent material that simultaneously represents the second electrode, protective dielectric layer 17, filler material 18, electrically-screening housing 19, a part of the housing representing a wire mesh and providing the access of the ambient atmosphere to the sensitive element 20, an air pump for a forced delivery of the ambient gas to the sensitive element of the device 21. (A) The capacitive sensor for the inward buckling with empty space between its two electrodes. (B) The inward-buckling capacitive sensor with a protective dielectric layer between its electrodes preventing their electric contact. (C) The outward-buckling capacitive sensor with the empty recess between its electrodes. (D) Modification of the outward-buckling capacitive hydrogen sensor in which the recess is filled with a material, preferentially possessing a high dielectric constant. (E) Combination of the capacitive hydrogen sensor with the electrically-screening housing when the HSM electrode represents a part of the said housing This combination may be used together with any type of the above capacitive sensors. (F) Another combination of the capacitive hydrogen sensor with the electrically-screening housing in which the both electrodes are not a part of the said housing, but at least a part of the housing represents an electrically-conductive mesh thus, providing access of the ambient gas to the sensitive element. This combination may be used together with any type of the above capacitive sensors. (G) A hydrogen capacitive sensor with the housing and pump for a forced delivery of the ambient gas to the sensitive element.

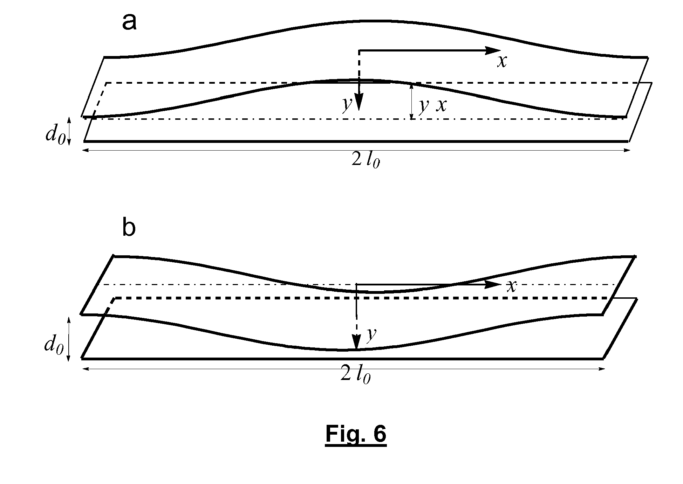

Figure 6 schematically shows two electrodes of the sensor under the outward buckling (a) and the inward buckling (b).

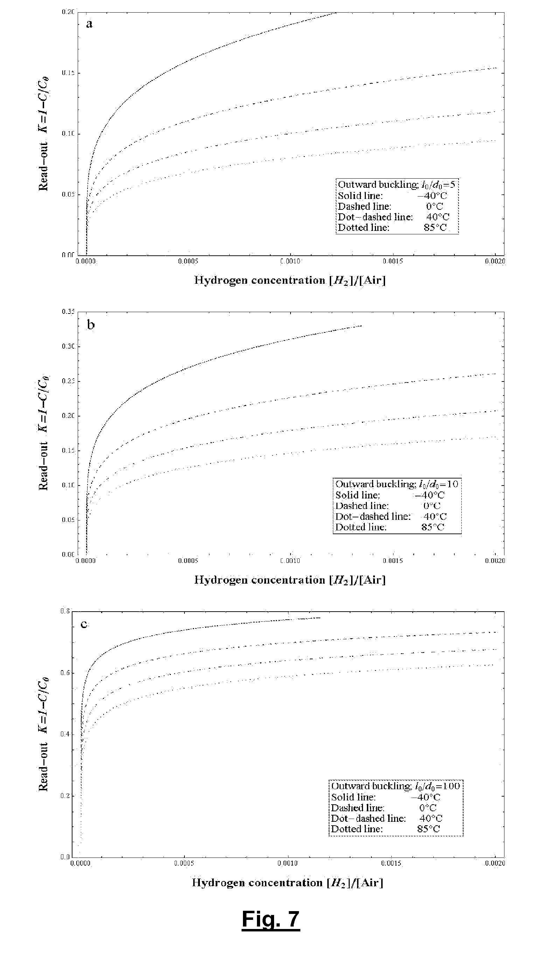

Figure 7 shows the read-out of the capacitive hydrogen sensor under the outward buckling at various temperatures and for three values of the aspect ratio: (a) $l_0/d_0$=5, (b) $l_0/d_0$=10 and (c) $l_0/d_0$=100.

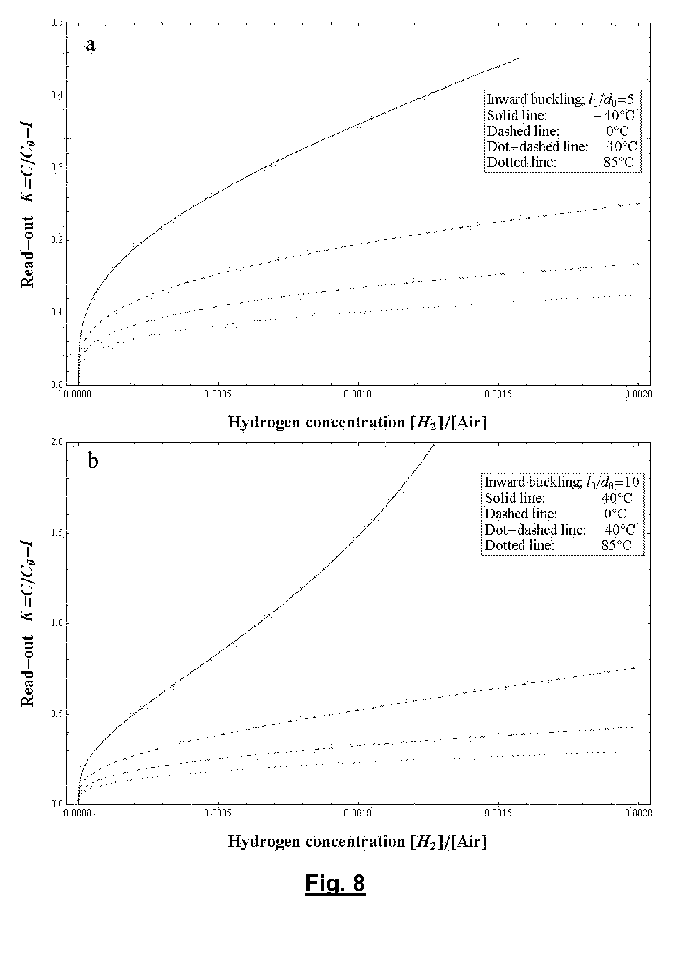

Figure 8 exhibits the read-out of the capacitive hydrogen sensor under the inward buckling at various temperatures and for two values of the aspect ratio: (a) $l_0/d_0$=5 and (b) $l_0/d_0$=10.

## Claims

1. A hydrogen sensor, comprising at least one reactive layer spanning at least along a first direction between two supporting points, said at least one reactive layer comprising an hydrogen-absorbent material, said material having a capability to expand upon absorption of hydrogen, and an assembly for detecting a deflection of said reactive layer, said deflection being caused by an expansion of said hydrogen-absorbent material in said first direction.

2. The hydrogen sensor as claimed in claim 1, wherein said reactive layer comprises a lamina of said hydrogen-absorbent material, and wherein said lamina of said hydrogen-absorbent material is fixed to said supporting points.

3. The hydrogen sensor as claimed in claim 1, wherein said reactive layer comprises a flexible carrier layer and a layer of hydrogen-absorbent material applied to said flexible carrier layer, and wherein said flexible carrier layer is fixed to said supporting points.

4. The hydrogen sensor as claimed in claim 3, wherein said flexible carrier layer comprises a polymer layer.

5. The hydrogen sensor as claimed in any one of claims 1 to 4, wherein said at least one reactive layer has a rectangular form with two shorter sides and two longer sides, and wherein each of said shorter sides of said reactive layer is fixed to one of said supporting points.

6. The hydrogen sensor as claimed in any one of claims 1 to 5, wherein said reactive layer further comprises a protective layer, said protective layer being arranged on the hydrogen-absorbent material.

7. The hydrogen sensor as claimed in any one of claims 1 to 6, wherein said assembly for detecting a deflection of said reactive layer comprises an optical system for determining reflection of a light beam at the deflected reactive layer.

8. The hydrogen sensor as claimed in any one of claims 1 to 6, wherein said assembly for detecting a deflection of said reactive layer comprises an electrode structure arranged in facing relationship with said hydrogen-absorbent material at a certain distance from said hydrogen-absorbent material and an associated electric circuit for detecting if an electrical contact is established between said hydrogen-absorbent material and said electrode structure due to the deflection of said reactive layer.

9. The hydrogen sensor as claimed in any one of claims 1 to 6, wherein said assembly for detecting a deflection of said reactive layer comprises a first electrode structure arranged on said reactive layer, a second electrode arranged in facing relationship with said first electrode structure at a certain distance from said first electrode structure, and an associated electric circuit for detecting if an electrical contact is established between said first electrode structure and said second electrode structure due to the deflection of said reactive layer.

10. The hydrogen sensor as claimed in any one of claims 1 to 6, wherein said assembly for detecting a deflection of said reactive layer comprises an electrode structure arranged at a certain distance from said hydrogen-absorbent material and an associated electric circuit for determining a variation of capacitance between said hydrogen-absorbent material and said electrode structure due to the deflection of said reactive layer.

11. The hydrogen sensor as claimed in any one of claims 1 to 6, wherein said assembly for detecting a deflection of said reactive layer comprises a first electrode structure arranged on said reactive layer, a second electrode arranged at a certain distance from said first electrode structure, and an associated electric circuit for determining a variation of capacitance between said first electrode structure and said second electrode structure due to the deflection of said reactive layer.

**Fig. 1**

**Fig. 2**

**Fig. 3**

**Fig. 4**

**Fig. 5A**

**Fig. 5B**

**Fig. 5C**

**Fig. 5D**

**Fig. 5E**

**Fig. 5F**

**Fig. 5G**

**Fig. 6**

**Fig. 7**

**Fig. 8**

Europäisches
Patentamt
European
Patent Office
Office européen
des brevets

## EUROPEAN SEARCH REPORT

Application Number

EP 08 16 5183

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | P.J. SHAVER: "Bimetal Strip Hydrogen Gas Detectors" THE REVIEW OF SCIENTIFIC INSTRUMENTS, vol. 40, no. 7, July 1969 (1969-07), pages 901-905, XP002516451 * page 903, left-hand column; figure 1 * * page 905, right-hand column * ----- | 1-3,5 | INV. G01N33/00 G01N27/22 B81B3/00 |
| Y | DE 195 36 719 A1 (DRESDEN EV INST FESTKOERPER [DE]) 3 April 1997 (1997-04-03) * the whole document * ----- | 1-3,5-7, 10,11 | |
| Y | BASELT D R ET AL: "Design and performance of a microcantilever-based hydrogen sensor" SENSORS AND ACTUATORS B, ELSEVIER SEQUOIA S.A., LAUSANNE, CH, vol. 88, no. 2, 15 January 2003 (2003-01-15), pages 120-131, XP004399080 ISSN: 0925-4005 * page 121, left-hand column * ----- | 1-3,10, 11 | TECHNICAL FIELDS SEARCHED (IPC) G01N B81B |
| Y | TIMOSHENKO S: "ANALYSIS OF BI-METAL THERMOSTATS" JOURNAL OF THE OPTICAL SOCIETY OF AMERICA, OPTICAL SOCIETY OF AMERICA, US, vol. 11, 1 January 1925 (1925-01-01), pages 233-256, XP009015501 ISSN: 0030-3941 * the whole document, see in particular Fig.4 and description thereof * ----- | 1-3,5-7, 10,11 | |
| A | WO 2008/035220 A (PHILIP MORRIS PROD [CH]) 27 March 2008 (2008-03-27) * page 7, line 33 - page 9, line 6; figure 10 * ----- | 1,7,10, 11 | |

The present search report has been drawn up for all claims

| Place of search | Date of completion of the search | Examiner |
|---|---|---|
| Munich | 23 February 2009 | Meyer, Fred |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 ..........................................................................

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 08 16 5183

This annex lists the patent family members relating to the patent documents cited in  the above-mentioned European search report.
The members are as contained in the European Patent Office EDP file on
The European Patent Office is in no way liable for these particulars which are merely given for the purpose of information.

23-02-2009

| Patent document cited in search report | | Publication date | Patent family member(s) | | Publication date |
|---|---|---|---|---|---|
| DE 19536719 | A1 | 03-04-1997 | NONE | | |
| WO 2008035220 | A | 27-03-2008 | US | 2008102532 A1 | 01-05-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **Simons, J. W. ; Flanagan, T. B.** Absorption isotherms of hydrogen in the .alpha.-phase of the hydrogen-palladium system. *Journal of Physical Chemistry,* 1965, vol. 69, 3773-3781
- Hydrogen in metals I and II. Springer-Verlag, 1978
- **Boulbich, A. A.** *J. Alloys Comp.,* 1993, vol. 196, 29
- *Physics and Mathematics, Moscow,* 1963
- *National Bureau of Standards,* 1964